# EUROPÄISCHE PATENTANMELDUNG

(11) **EP 3 733 637 A1**
(43) Veröffentlichungstag der Anmeldung: **04.11.2020**
(21) Anmeldenummer: 20159363.9
(22) Anmeldetag: 25.02.2020
(51) Int. Cl.: C07C 57/07, C07C 57/05, B01D 11/04, B01D 53/14

(54) **VERFAHREN ZUR RÜCKGEWINNUNG VON ACRYLSÄURE**

(71) Anmelder: BASF SE, 67056 Ludwigshafen am Rhein (DE)
(72) Erfinder: HAMMON, Ulrich, 67056 Ludwigshafen (DE); MUELLER-ENGEL, Klaus Joachim, 76297 Stutensee (DE); RISSEL, Steffen, 67056 Ludwigshafen (DE)
(74) Vertreter: Ellwanger, Arndt

(57) **Zusammenfassung**

Die vorliegende Erfindung betrifft ein Verfahren zur Rückgewinnung von Acrylsäure, welche durch katalytische Gasphasenoxidation von Propen erhalten wird,
wobei in einer Absorptionsstufe (K2) die Acrylsäure aus dem Reaktionsgemisch (1) der Gasphasenoxidation mit einem ersten Lösungsmittel (3) absorbiert und zur destillativen Reinigung abgezogen sowie ein Gasgemisch aus der Absorptionsstufe (K2) zu einer Kondensationsstufe weitergeleitet wird,
wobei in der Kondensationsstufe das Gasgemisch kondensiert, eine kondensierte Phase des Gasgemisches als Sauerwasser (4) abgezogen und einer Phasentrennoperation in einem Phasentrennbehälter (B1) unterzogen wird,
umfassend die Schritte
a) Zuführen der im Phasentrennbehälter (B1) gewonnenen wässrigen Phase (4*) des aus der Kondensationsstufe abgezogenen Sauerwassers (4) zu einer Extraktionsstufe (K7), in welcher in dem Sauerwasser (4) enthaltene Acrylsäure mit einem zweiten Lösungsmittel (5) extrahiert wird,
b) Zuführen des Acrylsäure enthaltenden Extrakts (6) zu einer Strippkolonne (K8), in welcher die Acrylsäure mit Kreisgas (8) aus dem zweiten Lösungsmittel (5) entfernt wird, wobei das abgetrennte zweite Lösungsmittel (5) nach Ausstrippung der Acrylsäure der Extraktionsstufe (K7) wieder zugeführt wird,
c) Zuführen des mit Acrylsäure beladenen Kreisgases (9) zu einem Strippkreisgaswäscher (K5), in welchem die Acrylsäure mit dem zu dem Strippkreisgaswäscher (K5) zugeführten ersten Lösungsmittelstrom (10) aus dem Kreisgas entfernt und in das erste Lösungsmittel (3) überführt wird, und
d) Zuführen des mit Acrylsäure beladenen ersten Lösungsmittels (3) zu einem ersten Teil zurück zu der Absorptionsstufe (K2).

Ferner betrifft die vorliegende Erfindung eine entsprechende Anlage zur Rückgewinnung von Acrylsäure

## Beschreibung

Die vorliegende Erfindung bezieht sich auf ein Verfahren zur Rückgewinnung von Acrylsäure, welche durch katalytische Gasphasenoxidation von Propen erhalten wird.

Verfahren zur Herstellung von Acrylsäure sowie deren Reinigung und Rückgewinnung sind aus dem Stand der Technik grundsätzlich bekannt. Acrylsäure ist aufgrund ihrer sehr reaktionsfähigen Doppelbindung sowie ihrer Säurefunktion ein wertvolles Monomer und Zwischenprodukt zur Herstellung von Polymerisaten, die für die unterschiedlichsten Produkte verwendet werden, beispielsweise absorbierende Harze, Bindemittel für wässrige Dispersionsfarben und dergleichen.

Acrylsäure wird im großen Maßstab durch eine heterogen katalysierte partielle Gasphasenoxidation von Propen und/oder Acrolein mit Sauerstoff oder Sauerstoff enthaltenden Gasen in Gegenwart von festen Katalysatoren und bei erhöhter Temperatur hergestellt. Um die Selektivität zu verbessern, wird die heterogen katalysierte partielle Oxidation von Propen zu Acrylsäure insbesondere in zwei aufeinanderfolgenden Schritten vorgenommen, wobei der erste Reaktionsschritt das Propen zu Acrolein umsetzt und der zweite Reaktionsschritt das Acrolein zu Acrylsäure, was häufig in zwei räumlich getrennten Reaktionsstufen durchgeführt wird. Hierbei wird keine reine Acrylsäure, sondern ein Produktgasgemisch erhalten, das neben dem Produkt Acrylsäure als Nebenkomponente nicht umgesetztes Acrolein und/oder Propen sowie Wasserdampf, Kohlendioxide, Stickstoff, Sauerstoff, Propan, Methan, niedrig gesättigte Carbonsäuren wie Ameisensäure, Essigsäure und Propionsäure, niedere Aldehyde wie Formaldehyd, Benzaldehyd und Furfurale sowie höhere Carbonsäuren bzw. deren Anhydride enthält.

Ein Teil dieser Nebenkomponenten ist leichter flüchtig als Acrylsäure (zum Beispiel Essigsäure) und wird auch als "Leichtsieder" bezeichnet. Ein anderer Teil ist schwerer flüchtig als Acrylsäure (zum Beispiel Phthalsäureanhydrid) und wird demgemäß als "Schwersieder" bezeichnet. Mit "Mittelsieder" werden Nebenkomponenten bezeichnet, die eine ähnliche Flüchtigkeit wie Acrylsäure aufweisen. Die Grundabtrennung der Acrylsäure aus dem gasförmigen Produktgasgemisch erfolgt in der Regel durch sorptive Verfahren, beispielsweise durch Gegenstromabsorption der Acrylsäure mit einem ersten Lösungsmittel bzw. Lösungsmittelgemisch.

Heute werden vier grundsätzliche Verfahrensvarianten für die Aufarbeitung unterschieden, die zum Erhalt des gereinigten Endprodukts führen.

Bei einer ersten Verfahrensvariante (die auch als "Extraktionsverfahren" bezeichnet werden kann) wird als Lösungsmittel Wasser eingesetzt, welches die Acrylsäure aus dem Gasstrom nach der Synthese auswäscht. Die weitere Abtrennung des Wassers erfolgt durch eine Extraktion und anschließende Destillationsschritte.

Eine zweite Verfahrensvariante (die auch als "Azeotropverfahren" bezeichnet werden kann) sieht vor, dass ebenfalls mit Wasser nach der Absorption gewaschen und die Wasserabtrennung durch eine Schleppmitteldestillation (azeotrope Destillation) und weitere Destillationsschritte durchgeführt wird.

Eine dritte Verfahrensvariante (die auch als "Kristallisationsverfahren" bezeichnet werden kann) besteht darin, das Reaktionsgas ohne Zusatz eines ersten Lösungsmittels fraktioniert zu kondensieren, wobei die weitere Aufarbeitung durch Kristallisation erfolgt.

Schließlich wird in einer vierten Verfahrensvariante (die auch als "Lösungsmittelverfahren" bezeichnet werden kann) die Acrylsäure mit einem hochsiedenden hydrophoben ersten Lösungsmittel aus dem Gasstrom aufgenommen. Diese Acrylsäureaufnahme erfolgt dabei selektiv, d.h. der im Gasstrom aus der Synthese enthaltene Wasserdampf verbleibt dabei weitgehend in der Gasphase.

Diese unterschiedlichen vier Verfahrensvarianten unterscheiden sich sowohl durch die erhaltene Produktqualität und den notwendigen Energieeinsatz als auch durch die Art und Zusammensetzung der Stoffströme, welche aus dem Verfahren abgezogenen werden und welche die abgetrennten Nebenkomponenten erhalten.

Die vierte Verfahrensvariante wird beispielsweise in WO 2011/000808 A2 in Form eines Verfahrens zur Abtrennung von Acrylsäure beschrieben, konkret eines Verfahrens zur Abtrennung von Acrylsäure aus dem Produktgasgemisch einer heterogen katalysierten partiellen Gasphasenoxidation wenigstens einer C3-Vorläuferverbindung zu Acrylsäure, das neben Acrylsäure, Wasserdampf und Glyoxal noch von den vorgenannten Verbindungen verschiedene Leichtsieder, Mittelsieder, Schwersieder und Schwerkondensierbare als Nebenbestandteile enthält. Bei diesem Verfahren wird das Produktgasgemisch in einem Direktkühler durch Direktkühlung mit einer feinteilig versprühten Kühlflüssigkeit abkühlt, wobei eine Teilmenge der Kühlflüssigkeit verdampft. Das abgekühlte Produktgasgemisch wird zusammen mit verdampfter und nicht verdampfter Kühlflüssigkeit in den Sumpfraum einer Absorptionskolonne geführt, der mit dem darüber befindlichen, trennwirksame Einbauten aufweisenden, Absorptionsraum der Absorptionskolonne durch einen zwischen beiden befindlichen Kaminboden KB1, der wenigstens einen Kamin aufweist, verbunden ist, und von dem aus das abgekühlte Produktgasgemisch und verdampfte Kühlflüssigkeit durch den wenigsten einen Kamin des Kaminbodens KB1 hindurch in den Absorptionsraum strömt und in selbigem zu einem in diesem absteigenden hochsiedenden (ersten) Absorptionsmittel im Gegenstrom aufsteigt. Dabei läuft auf dem Kaminboden KB1 Acrylsäure in Absorptionsmittel absorbiert enthaltendes Absorbat A auf. Vom Kaminboden KB1 wird auf selbigem auflaufendes, Acrylsäure in Absorptionsmittel absorbiert enthaltendes, Absorbat A aus der Absorptionskolonne herausgeführt, eine Teilmenge an aus der Absorptionskolonne herausgeführtem Absorbat A unter Ausbildung einer im Sumpfraum befindlichen Sumpfflüssigkeit dem Sumpfraum der Absorptionskolonne zugeführt sowie gegebenenfalls eine andere Teilmenge des Absorbats A abgekühlt und oberhalb des Kaminbodens KB1 in die Absorptionskolonne rückgeführt. Aus der verbliebenen Restmenge RA an aus der Absorptionskolonne herausgeführtem Absorbat A werden gegebenenfalls in einer Strippeinheit Leichtsieder ausgestrippt und dabei ein an Leichtsiedern entreichertes Absorbat A* erzeugt. Die Restmenge RA an Absorbat A oder das Absorbat A* wird einer Rektifikationskolonne mit Verstärkungs- und Abtriebsteil zugeführt, im Abtriebsteil der Rektifikationskolonne das Absorptionsmittel angereichert sowie aus dem Abtriebsteil Absorptionsmittel mit einem Acrylsäuregewichtsanteil von < 1 Gew.-% herausgeführt, im Verstärkungsteil der Rektifikationskolonne die Acrylsäure angereichert und aus dem Verstärkungsteil eine rohe Acrylsäure P mit einem Gewichtsanteil der Acrylsäure von > 90 Gew.-% herausgeführt. Aus dem Sumpfraum der Absorptionskolonne wird Absorptionsmittel enthaltende Sumpfflüssigkeit entnommen, eine Teilmenge an dieser entnommenen Sumpfflüssigkeit als Kühlflüssigkeit dem Direktkühler zugeführt und die Restmenge an dieser entnommenen Sumpfflüssigkeit einer Destillationseinheit zugeführt, die eine Destillationskolonne und einen Umlaufwärmeaustauscher umfasst, in der Destillationskolonne die der Destillationseinheit zugeführte Sumpfflüssigkeit destillativ in Brüden, dessen Gewichtsanteil an Absorptionsmittel größer ist als der Gewichtsanteil der Sumpfflüssigkeit an Absorptionsmittel, und in flüssiges Konzentrat, dessen Gewichtsanteil an höher als das Absorptionsmittel siedenden Bestandteilen B größer ist, als der Gewichtsanteil der Sumpfflüssigkeit an Bestandteilen B, aufgetrennt. Ein Brüdenstrom, gegebenenfalls nach in einem indirekten Wärmeaustauscher erfolgter Abkühlung und/oder Kondensation desselben, wird oberhalb des Kaminbodens KB1 in die Absorptionskolonne rückgeführt, und am unteren Ende der Destillationskolonne wird ein Mengenstrom M des dort in einer Standhöhe S flüssig auflaufenden Konzentrats mit der Temperatur T1 aus der Destillationskolonne herausgeführt. Ein Teilmengenstrom TAu von diesem Mengenstrom M aus dem Verfahren der Abtrennung von Acrylsäure wird aus dem Produktgemisch ausgeschleust und der Reststrom RM des Mengenstroms M über den Umlaufwärmeaustauscher mit der Temperatur T2 > T1 oberhalb der Entnahme des Mengenstroms M aus der Destillationskolonne in die Destillationskolonne zurückgeführt. Die mittlere Verweilzeit tv der Bestandteile des Teilmengenstroms TAu beträgt in der Destillationseinheit < 40 h.

Die dritte Verfahrensvariante wird beispielsweise in DE 103 32 758 A1 oder WO 2015/091628 A1 offenbart. Bei dieser Verfahrensvariante wird das Reaktionsgas wie in der vierten Verfahrensvariante durch Eindüsen und anschließendes Verdampfen einer Kühlflüssigkeit abgekühlt und danach in eine Kondensationskolonne geleitet, die trennwirksame Einbauten enthält. In dieser Kondensationskolonne wird die Acrylsäure durch den Rücklauf der Absorptionskolonne aufgenommen, wobei weiterhin leichtsiedende und schwersiedende Verbindungen im Kopf und im Sumpfbereich der Kondensationskolonne angereichert werden. Im unteren Drittel der Kondensationskolonne wird eine mehr als 90 Gew.-% enthaltene Acrylsäure zur weiteren Reinigung in eine anschließende Kristallisationsanlage geleitet. Aus der Kristallisationsanlage wird das Produkt Acrylsäure in einer Reinheit > 99 Gew.-% abgezogen. Die in der Kristallisationsanlage abgetrennten Verunreinigungen werden als sog. "Mutterlauge" in die Kondensationskolonne zur weiteren Verarbeitung zurückgeführt. Am Sumpf der Absorptionskolonne wird eine Schwersiederfraktion abgezogenen, die in eine Spaltkolonne überführt wird, wo oligomere Acrylsäure in monomere Acrylsäure gespalten und die abgetrennte Acrylsäure gasförmig in die Kondensationskolonne zurückgeführt wird. Der Schwersiederrückstand wird flüssig aus dem Sumpf der Spaltkolonne abgezogen und einer Entsorgungseinheit zugeführt. Am Kopf der Kondensationskolonne wird das aus dem trennwirksamen Bereich der Absorptionskolonne austretende Gas in einem Kondensationsteil gekühlt und dort kondensierbare Nebenkomponenten, im Wesentlichen Reaktionswasser und Essigsäure wie nicht abgetrennte Acrylsäure, auskondensiert. Das so gewonnene sog. "Sauerwasser" wird am Kopf der Kolonne über eine Extraktionseinheit in eine Entsorgungseinheit überführt. In der Extraktionseinheit wird die im Sauerwasser enthaltene Acrylsäure abgetrennt und in die Absorptionskolonne zurückgeführt.

Der dritten und der vierten Verfahrensvariante ist gemein, dass nach der Abtrennung der Acrylsäure vom Reaktionswasser weiterhin Acrylsäure im Sauerwasser verbleibt. In der vierten Verfahrensvariante wurde die Acrylsäure, die mit ca. 4 Gew.-% in dem Sauerwasser enthalten ist verworfen, und zusammen mit anderen organischen Nebenkomponenten, die im Sauerwasser enthalten sind, in einer thermischen Behandlungsanlage verbrannt. Mit gestiegenen Kosten für Rohstoffe und Energie sowie im Hinblick auf Umweltaspekte ist es jedoch inzwischen wirtschaftlich interessant, den im Sauerwasser verbliebenen Rest an Acrylsäure ebenfalls rückzugewinnen. Demgegenüber war der Anteil von Acrylsäure im Sauerwasser, welches in der dritten Verfahrensvariante erhalten wird, mit 8 Gew.-% bis 15 Gew.-% bereits grundsätzlich höher, so dass hier eine Rückgewinnung der Acrylsäure beispielsweise durch Extraktion der Acrylsäure aus dem Sauerwasser rentabel war.

Nachteilig an der Aufarbeitung der vierten Verfahrensvariante, insbesondere gegenüber der dritten Verfahrensvariante, ist der deutlich erhöhte Verlust an wertvollem Produkt Acrylsäure, das bislang nur unzureichend aus dem Sauerwasser extrahiert wird.

Der vorliegenden Erfindung liegt daher die objektiv-technische Aufgabe zugrunde, ein Verfahren zur Rückgewinnung von Acrylsäure anzugeben sowie eine entsprechende Anlage bereitzustellen, womit der Anteil der im Sauerwasser verbleibenden Acrylsäure deutlich verringert werden kann, um so die Gesamtausbeute bei der Acrylsäureherstellung, insbesondere unter Anwendung der Aufarbeitung nach der vierten Verfahrensvariante, zu verbessern.

Diese Aufgabe wird im ersten Aspekt der vorliegenden Erfindung durch ein Verfahren zur Rückgewinnung von Acrylsäure gelöst, welche durch katalytische Gasphasenoxidation von Propen erhalten wird,
wobei in einer Absorptionsstufe (K2) die Acrylsäure aus dem Reaktionsgemisch (1) der Gasphasenoxidation mit einem ersten Lösungsmittel (3) absorbiert und zur destillativen Reinigung abgezogen sowie ein Gasgemisch aus der Absorptionsstufe (K2) zu einer Kondensationsstufe weitergeleitet wird,
wobei in der Kondensationsstufe das Gasgemisch kondensiert, eine kondensierte Phase des Gasgemisches als Sauerwasser (4) abgezogen und einer Phasentrennoperation in einem Phasentrennbehälter (B1) unterzogen wird, umfassend die Schritte:
a) Zuführen der im Phasentrennbehälter (B1) gewonnenen wässrigen Phase (4*) des aus der Kondensationsstufe abgezogenen Sauerwassers (4) zu einer Extraktionsstufe (K7), in welcher in dem Sauerwasser (4) enthaltene Acrylsäure mit einem zweiten Lösungsmittel (5) extrahiert wird,
b) Zuführen des Acrylsäure enthaltenden Extrakts (6) zu einer Strippkolonne (K8), in welcher die Acrylsäure mit Kreisgas (8) aus dem zweiten Lösungsmittel (5) entfernt wird, wobei das abgetrennte zweite Lösungsmittel (5) nach Ausstrippung der Acrylsäure der Extraktionsstufe (K7) wieder zugeführt wird,
c) Zuführen des mit Acrylsäure beladenen Kreisgases (9) zu einem Strippkreisgaswäscher (K5), in welchem die Acrylsäure mit dem zu dem Strippkreisgaswäscher (K5) zugeführten ersten Lösungsmittelstrom (10) aus dem Kreisgas (8) entfernt und in das erste Lösungsmittel (3) überführt wird, und
d) Zuführen des mit Acrylsäure beladenen ersten Lösungsmittels (3) zu einem ersten Teil zurück zu der Absorptionsstufe (K2).

Aufgrund der unterschiedlichen Verfahrensführungen der dritten und der vierten Verfahrensvariante wird in der vierten Verfahrensvariante ein Sauerwasserstrom (4 bzw. 4*) erhalten, der neben den leichtsiedenden kondensierbaren Nebenkomponenten wie in der dritten Verfahrensvariante auch schwersiedende Nebenkomponenten, Diacrylsäure und Reste von Lösungsmittelbestandteilen erhalten. Daher war es bislang als nicht sinnvoll und nicht machbar erachtet worden, eine zusätzliche Extraktion des Sauerwassers, wie sie in der dritten Verfahrensvariante durchgeführt wird, auf die vierte Verfahrensvariante zu übertragen.

Im Rahmen der vorliegenden Erfindung wurde jedoch überraschenderweise festgestellt, dass eine zusätzliche Extraktion von Acrylsäure aus dem Sauerwasser mit entsprechenden Anpassungen auch in der vierten Verfahrensvariante sinnvoll und anwendbar ist und so zu deutlich höheren Ausbeuten des Produkts Acrylsäure führen kann.

Das erfindungsgemäße Verfahren weist daher den Vorteil auf, dass durch das Vorsehen einer zusätzlichen Sauerwasserextraktion mit anschließender Lösungsmittelbehandlung der Anteil der in Sauerwasser, welches der Entsorgung zugeführt wird, enthaltenen Acrylsäure deutlich verringert werden kann. Insbesondere kann der Anteil der Acrylsäure auf unter 1 Gew.-%, bezogen auf das Gesamtgewicht des Abwasserstroms (11), verringert werden. Hierdurch kann gleichzeitig die Gesamtausbeute an Acrylsäure, bezogen auf das Gesamtverfahren der vierten Verfahrensvariante, um bis zu 2 % verbessert werden.

In einer Weiterbildung des erfindungsgemäßen Verfahrens ist der Extraktionsstufe (K7) eine Vorextraktionsstufe vorgeschaltet, in welcher dem Sauerwasser (4) ein Teilstrom (12) des aus dem Strippkreisgaswäscher (K5) austretenden ersten Lösungsmittelstroms zugeführt wird, nach Phasentrennung im Phasentrennbehälter (B1) das abgetrennte erste Lösungsmittel (3) der Absorptionsstufe (K2) zugeführt wird und das im Phasentrennbehälter (B1) abgetrennte Sauerwasser (4*) nach der Vorextraktion in der Vorextraktionsstufe zur Extraktionskolonne (K7) eingeleitet wird.

Mit der der Extraktionsstufe (K7) vorgelagerten Vorextraktion kann neben der Entfernung von Lösungsmittel aus dem Sauerwasser (4) insbesondere der Anteil an Diacrylsäure im ersten Lösungsmittelstrom (10) signifikant verringert werden, insbesondere auf einen Wert von unter 1,5 %, bezogen auf das Gesamtgewicht des ersten Lösungsmittelstroms (10). Diese dimere Acrylsäure wird in den Sauerwasserstrom (4) aufgenommen und der Extraktionsstufe (K7) zugeführt.

Um eine effizientere Abtrennung von leichtsiedenden Nebenkomponenten wie Essigsäure, Acetaldehyd, Formaldehyd, Acrolein und Glyoxal zu ermöglichen, hat es sich als vorteilhaft herausgestellt, wenn die Absorptionsstufe (K2) mit einer Zulauftemperatur von 45 °C bis 65 °C und einer Ablauftemperatur von 100 °C bis 125 °C betrieben wird, wodurch das aus der Kondensationsstufe abgezogene Sauerwasser (4) neben einem erhöhten Gehalt an Acrylsäure ferner Diacrylsäure enthält.

Durch die Erhöhung der Temperatur wird die Bildung bzw. Verschleppung von Glyoxal verhindert, welches eine polymerisationsfördernde Komponente darstellt, welche die Reinheit der Acrylsäure herabsetzt. Der Vorteil der geringeren Glyoxalmenge bedingt einen höheren Anteil von Acrylsäure im Sauerwasser sowie eine verstärkte Bildung von Diacrylsäure.

Einer der Gründe, warum eine Extraktion in der vierten Verfahrensvariante mit der vorliegenden Erfindung wirtschaftlich wird, ist, dass die Absorptionsstufe (K2) bei höherer Temperatur betrieben werden kann. Die erhöhte Temperatur bewirkt eine effektivere Abtrennung von leichtsiedenden Nebenkomponenten, wodurch das Foulingverhalten von nachgeschalteten Wärmetauschern und Trennapparaten und die Produktqualität verbessert werden. Durch die Fahrweise bei erhöhter Temperatur in der Absorptionsstufe (K2) der vierten Verfahrensvariante steigt die Anlagenverfügbarkeit, weil nachgelagerte Anlagenteile langsamer verschmutzen und somit längere Zyklen ohne Abstellung zur Reinigung gefahren werden können. Weiterhin fällt der Energieverbrauch, weil weniger leichtsiedende Komponenten in den der Absorption nachfolgenden Trennoperationen abgetrennt werden müssen, um die Zielqualität des Produkts zu erreichen. Allerdings steigt hierbei jedoch auch der Anteil an Acryl- und Diacrylsäure im Sauerwasser an. Somit ergibt sich also zusätzlich zur längeren Standzeit ein höheres Rückgewinnungspotential an Acrylsäure aus dem Sauerwasser.

In einer speziellen Weiterbildung dieser Ausführungsform wird die Diacrylsäure neben der Acrylsäure selektiv extrahiert, in Schritt b) in der Strippkolonne (K8) aufgespalten und auf diese Weise als monomere Acrylsäure rückgewonnen.

Dadurch erhöht sich die Gesamtausbeute der vierten Verfahrensvariante um weitere 0,5 % bis 1 %.

Gemäß einer Weiterbildung ist dem Strippkreisgaswäscher (K5) im Strom des ersten Lösungsmittels (3) eine Kühleinrichtung (W1) vorgeschaltet, mit welcher das erste Lösungsmittel (3) vorgekühlt wird, um den erhöhten Energieeintrag durch die Einleitung des Strippkreisgases aus der Strippkolonne (K8) der Sauerwasserextraktion gegen zu kompensieren.

Durch die höhere Temperatur des dem Strippkreisgaswäscher (K5) zugeführten Gasstroms würden die Temperaturbedingungen in dieser Kolonne stark erhöht und damit die beabsichtigte Waschwirkung des Kreisgases in der Kolonne stark verringert werden. Durch das Vorsehen der Kühleinrichtung (W1) kann die Temperatur in der Strippkreisgaskolonne (K5) auf dem gewünscht niedrigem Niveau von 30 °C bis 50 °C gehalten und damit die gewünschte Waschwirkung ohne Einbußen eingestellt werden.

Es hat sich für das Erreichen einer möglichst hohen Rückgewinnungsrate von Acrylsäure und Diacrylsäure als vorteilhaft herausgestellt, wenn die Strippkolonne (K8) bei einer Temperatur von mindestens 170 °C im Sumpf der Kolonne betrieben wird, bevorzugt bei 180 °C, besonders bevorzugt bei 190 °C, und die Verweilzeit der dem Sumpfkreislauf der Strippkolonne (K8) zugeführten Flüssigkeit > 0,5 h besonders bevorzugt > 1 h beträgt.

In einer speziellen Ausführungsform umfasst das erste Lösungsmittel ein Gemisch von Diphyl und Dimethylphthalat und das zweite Lösungsmittel Dimethylphthalat oder Diethylphthalat. Unter "Diphyl" wird gemäß der vorliegenden Erfindung ein Diphenyloxid/Diphenyl-Eutektikum um Verhältnis 3:1 verstanden. Ein Gemisch umfassend Diphyl und Dimethylphthalat hat sich als erstes Lösungsmittel bzgl. der Absorptionsfähigkeit von Acrylsäure bei gleichzeitig geringem Foulingverhalten bei der Absorption bewährt. Dimethylphthalat und Diethylphthalat zeichnen sich durch eine hohe selektive Extraktionsfähigkeit bzgl. Acrylsäure in einem Stoffgemisch von Wasser und anderen organischen Säuren, wie beispielsweise Ameisensäure und Essigsäure, aus, wobei das Diethylphthalat sich besonders eignet, da es eine sehr geringe Löslichkeit im Raffinat nach der Extraktion bei hoher Selektivität bzgl. Acrylsäure neben niedrigen Carbonsäuren wie Essigsäure und Ameisensäure aufweist.

Es hat sich für eine möglichst quantitative Extraktion als vorteilhaft herausgestellt, wenn diese in der Extraktionskolonne (K7) bei Temperaturen von < 80 °C, bevorzugt 40 °C durchgeführt wird, um die Verluste des Extraktionsmittels (zweites Lösungsmittel) gering zu halten.

Wenn in der nachstehenden Beschreibung der erfindungsgemäßen Anlage Verfahrensmerkmale genannt werden, so beziehen sich diese insbesondere auf das erfindungsgemäße Verfahren. Ebenso beziehen sich gegenständliche Merkmale, die in der vorstehenden Beschreibung des erfindungsgemäßen Verfahrens angeführt werden, auf die erfindungsgemäße Anlage.

Die vorstehend genannte objektiv-technische Aufgabe wird im zweiten Aspekt der vorliegenden Erfindung durch eine Anlage zur Rückgewinnung von Acrylsäure gelöst, welche eine Absorptionsstufe (K2) zur Absorption von Acrylsäure aus dem Reaktionsgemisch einer Gasphasenoxidation und eine daran anschließende Kondensationsstufe zur Kondensation des Gasgemischs als Sauerwasser (4) aufweist, ferner umfassend
- eine Extraktionsstufe (K7) zur Extraktion der in dem Sauerwasser (4) enthaltene Acrylsäure mit einem zweiten Lösungsmittel (5), wobei die Extraktionsstufe (K7) der Kondensationsstufe (K2) nachgelagert und mit dieser über eine Sauerwasser-Leitung verbunden ist,
- eine Strippkolonne (K5) zum Entfernen der Acrylsäure mit Kreisgas (13) aus dem zweiten Lösungsmittel (5), wobei die Strippkolonne (K8) der Extraktionsstufe (K7) nachgelagert und mit dieser über eine Extrakt-Leitung verbunden ist,
- eine Lösungsmittel-Leitung, welche die Strippkolonne (K8) und die Extraktionsstufe (K7) verbindet, zum Rückführen des zweiten Lösungsmittels (5) in die Extraktionsstufe (K7),
- einen Sumpfwärmetauscher (W3) im Sumpfkreislauf der Strippkolonne (K8) zum Bereitstellen der für das nahezu vollständige Ausstrippen der Acrylsäure aus dem Extraktstrom benötigten Energie,
- einen Strippkreisgaswäscher (K5) zum Entfernen der Acrylsäure mit einem zu dem Strippkreisgaswäscher (K5) zugeführten ersten Lösungsmittelstrom (10) aus dem Kreisgas, wobei der Strippkreisgaswäscher (K5) der Strippkolonne (K8) nachgelagert und mit dieser über eine Kreisgas-Leitung (9) verbunden ist, und
- eine erste Leitung zum Zuführen des mit Acrylsäure beladenen ersten Lösungsmittels (3) zu einem ersten Teil (zurück zu der Absorptionsstufe (K2).

Die erfindungsgemäße Anlage ist insbesondere dazu ausgelegt, das vorstehend beschriebene erfindungsgemäße Verfahren durchzuführen.

Die erfindungsgemäße Anlage weist grundsätzlich die gleichen Vorteile wie das erfindungsgemäße Verfahren, nämlich dass durch das Vorsehen einer zusätzlichen Sauerwasserextraktion (Extraktionsstufe (K7)) mit anschließender Lösungsmittelbehandlung (Strippkolonne (K8)) der Anteil der in Sauerwasser, welches der Entsorgung zugeführt wird, enthaltenen Acrylsäure deutlich verringert werden kann.

Aufgrund der unterschiedlichen Verfahrensführungen in der vierten Verfahrensvariante gegenüber der dritten Verfahrensvariante war es bisher aufgrund der im Sauerwasser der vierten Verfahrensvariante vorhandenen schwersiedenden Nebenkomponenten wie Maleinsäure, Phthalsäure und Resten von erstem Lösungsmittel als nicht sinnvoll und nicht machbar erachtet worden, eine zusätzliche Extraktionsstufe für das Sauerwasser, wie sie prinzipiell in der dritten Verfahrensvariante vorhanden ist, in das Verfahrenskonzept der vierten Verfahrensvariante zu übertragen.

Im Rahmen der vorliegenden Erfindung wurde jedoch überraschenderweise festgestellt, dass das Vorsehen einer zusätzlichen Extraktionsstufe (K7) für die Acrylsäure aus dem Sauerwasser mit entsprechend überarbeiteten Auslegungen auch in einer angepassten Anlage für die vierte Verfahrensvariante vier sinnvoll und anwendbar ist und so zu deutlich höhere Ausbeuten des Produkts Acrylsäure führen kann.

In einer Weiterbildung der erfindungsgemäßen Anlage umfasst diese ferner eine Vorextraktionsstufe, die in der Sauerwasser-Leitung vor der Extraktionsstufe (K7) angeordnet ist.

Das Vorsehen der erfindungsgemäßen Vorextraktion ermöglicht neben der Entfernung von Lösungsmittel aus dem Sauerwasser insbesondere eine signifikante Verringerung des Anteils an Diacrylsäure im Strom des ersten Lösungsmittels (3).

Eine andere Ausführungsform der erfindungsgemäßen Anlage sieht vor, dass diese ferner eine Kühleinrichtung (W1) zum Vorkühlen des ersten Lösungsmittelstroms (10) umfasst, wobei die Kühleinrichtung (W1) vor dem Strippkreisgaswäscher (K5) angeordnet ist.

Durch das Vorsehen der Kühleinrichtung (W1) kann eine negative Beeinflussung des Strippkreisgaswäschers (K5) durch die höhere Temperatur des zugeführten Gasstroms deutlich reduziert werden.

Es ist ferner bevorzugt, wenn in der erfindungsgemäßen Anlage ein einen Kreuzwärmetauscher (W2) vorgesehen ist zum Erwärmen des Extraktstroms vor Zulauf in die Strippkolonne (K8) bei gleichzeitigem Abkühlen des abgereicherten Extraktionsmittelstroms aus der Extraktionskolonne (K7).

Eine andere Weiterbildung sieht vor, dass in der erfindungsgemäßen Anlage ein Wärmetauscher (W4) zum Abkühlen des Extraktionsmittelstroms vor Eintritt in die Extraktionskolonne (K7) vorgesehen ist. Dieser Wärmetauscher (W4) dient zur Begrenzung der Verluste des zweiten Lösungsmittels über das Raffinat.

Weitere Ziele, Merkmale, Vorteile und Anwendungsmöglichkeiten ergeben sich aus der nachfolgenden Beschreibung von die Erfindung nicht einschränkenden Ausführungsbeispielen anhand der Figuren. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbeziehung. Es zeigen:
- Fig. 1: eine schematische Darstellung des erfindungsgemäßen Teils einer Anlage zur Rückgewinnung von Acrylsäure,
- Fig. 2: ein Fließbild des erfindungsgemäßen Teils der in Fig. 1 dargestellten Anlage und
- Fig. 3: ein Diagramm zum Einfluss der Absorptionstemperatur auf den Acrylsäureverluste.

Sowohl technische wie auch wirtschaftliche Faktoren beeinflussen die Wirtschaftlichkeit der Rückgewinnung von Acrylsäure. Zu den technischen Faktoren zählen, dass eine Temperaturanhebung in der Absorptionsstufe K2 die Laufzeit der Anlage verlängert, gleichzeitig aber auch die Acrylsäurekonzentration im Sauerwasser erhöht. Zu den wirtschaftlichen Faktoren gehört insbesondere der Anstieg des Propen-Preises in den vergangenen Jahren um 40 %. Das Sauerwasser enthält vorliegend ca. 2,5 % der gesamten Acrylsäureproduktion und geht nach dem Stand der Technik direkt zur Verbrennung. Da der wirtschaftliche Anreiz inzwischen recht hoch ist, konservativ geschätzt beträgt das wirtschaftliche Potential 2 bis 4 Millionen € pro Jahr und Anlage, bringt die vorliegende Erfindung enorme Vorteile.

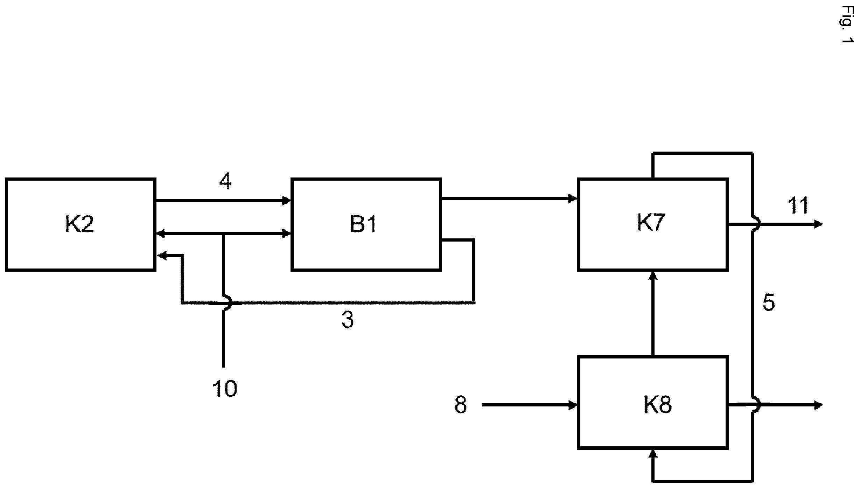

In Figur 1 wird schematisch das Grundprinzip des relevanten Teils einer erfindungsgemäßen Anlage zur Durchführung des erfindungsgemäßen Verfahrens dargestellt. Sauerwasser aus Absorption und Kondensation, d.h. aus der Absorptionsstufe K2 und der Kondensationsstufe, wird zunächst der Vorextraktionsstufe zugeführt und dann in die Extraktionsstufe K7 geleitet. In der Vorextraktionsstufe wird das erste Lösungsmittel Diphyl entfernt und der Absorptionsstufe K2 wieder zugeführt. In der Extraktionsstufe K7 wird das die Acrylsäure enthaltende zweite Lösungsmittel Dimethylphthalat aus dem Sauerwasser entfernt und über die Strippkolonne K8 im Kreis zur Extraktionsstufe K7 zurückgeführt. Die in der Strippkolonne K8 entfernte Acrylsäure wird dem Strippkreisgaswäscher K5 zugeleitet.

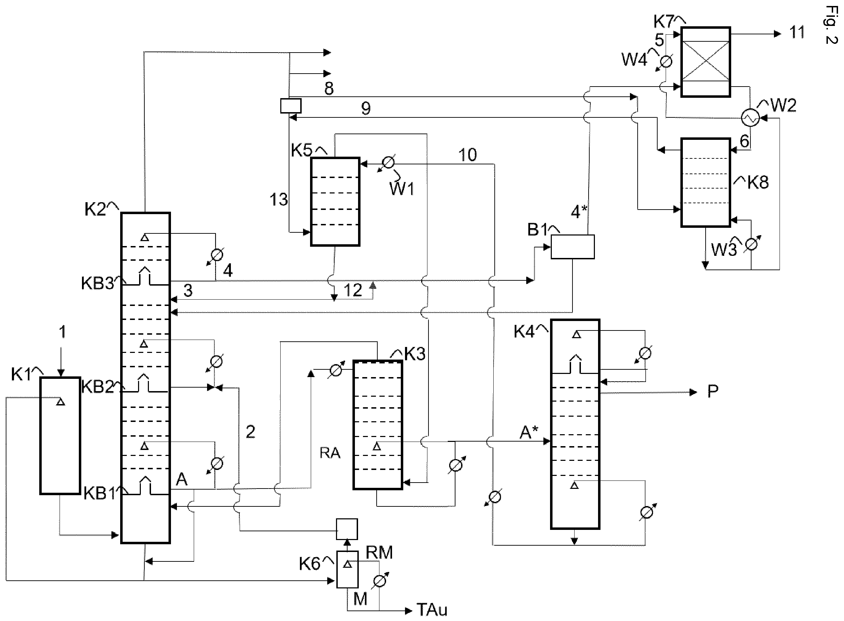

Figur 2 zeigt ein Fließbild des erfindungsgemäßen Teils der in Fig. 1 dargestellten erfindungsgemäßen Anlage, welche in ihrer Grundstruktur einer Anlage der vierten Verfahrensvariante entspricht, jedoch erfindungsgemäß eine Sauerwasserextraktion in der Extraktionsstufe K7 mit anschließender Lösungsmittel-Rückgewinnung und Acrylsäure-Abtrennung in der Strippkolonne K8 aufweist.

Die Extraktionskolonne K7 hat bevorzugt eine strukturierte Packung als trennwirksame Einbauten und besonders bevorzugt eine Packung, die durch Trennbleche in einzelne Segmente (wie in der WO 2015/091628 A1 beschrieben) unterteilt ist.

Die Strippkolonne K8 wird in der bevorzugten Version mit Trennböden (DualFlow, Thormann, Sieb- oder Glockenböden) bestückt.

Als Sumpfwärmetauscher W3 der Strippkolonne K8 wird bevorzugt eine Zwangsumlaufsentspannungsverdampfer eingesetzt.

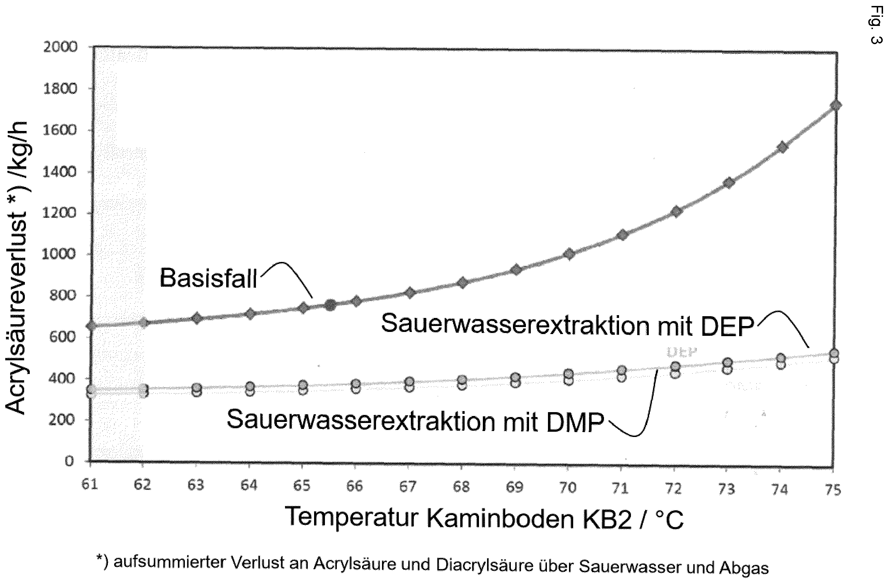

In Figur 3 ist dargestellt, wie die Acrylsäure-Verluste mit steigender Temperatur im Lösungsmittelzulauf ansteigen und wie weit diese Verluste mittels Extraktion reduziert werden können. Hier sind im Vergleich als Lösungsmittel Diethylphthalat (DEP) und Dimethylphthalat (DMP) dargestellt, wobei Dimethylphthalat besonders bevorzugt ist, da es bereits fester Bestandteil des ersten Lösungsmittels ist.

Die Verfahrensbedingungen der Aufarbeitung nach der dritten Verfahrensvariante haben unter anderem den Vorteil, dass nahezu keine Diacrylsäure im Sauerwasseraustrag der Kondensationskolonne vorhanden ist. Demgegenüber wird in der vierten Verfahrensvariante eine signifikante Menge an Diacrylsäure gebildet, bzw. gelangt durch die Vorextraktion in das aus der Absorptionsstufe K2 abgezogenen Sauerwasser 4. Da dieses Wasser direkt einer thermischen Entsorgung zugeführt wird, stellt dieser Anteil an Diacrylsäure in dem Sauerwasser 4 einen direkten Verlust an Acrylsäure dar.

Ziel dieser Vorextraktion war es, die mittelsiedenden und schwersiedenden Nebenkomponenten aus dem ersten Lösungsmittel abzutrennen, nicht jedoch Acrylsäure rückzugewinnen. Hier wurden insbesondere die Anhydride von Phthalsäure und Maleinsäure abgetrennt. Bei dieser Verfahrensführung wurde jeweils eine Teilmenge des Lösungsmittels über die Vorextraktion gefahren. In diesem Lösungsmittel befindet sich in geringen Anteilen (0,5 Gew.-% - 1,5 Gew.-%) Diacrylsäure, welche durch die Vorextraktion in das Sauerwasser weitgehend überführt wird. Nach der vierten Verfahrensvariante wurde auch dieser nicht unerhebliche Anteil an dimerisiertem Produkt dem Verfahren entzogen und als Abfall verworfen.

Nachfolgend wird nun das erfindungsgemäße Verfahren näher beschrieben.

Ziel der vorliegenden Erfindung war es, die Wirtschaftlichkeit der vierten Verfahrensvariante zur Aufarbeitung auch bei erhöhten Zulauftemperaturen des ersten Lösungsmittels zum Absorber zu erhöhen. Erhöhte Zulauftemperaturen bedeuten hier Temperaturen > 50 °C.

In der erfindungsgemäßen Extraktionsstufe K7 für das Sauerwasser 4 wird am Boden der Sauerwasserstrom 4* zugeführt und mit dem Strom des zweiten Lösungsmittels 5, vorzugsweise Dimethylphthalat (DMP), in Kontakt in einer Gegenstromextraktion gebracht. Dabei nimmt das Dimethylphthalat in hoher Selektivität die Acrylsäure (und Diacrylsäure) auf und wird der Extraktionsstufe K7 als Strom 6 entzogen. Am Kopf der Extraktionsstufe K7 wird das Raffinat 11 abgeführt, was im Wesentlichen aus Wasser besteht und geringe Mengen Essigsäure, Ameisensäure, Phthalsäure und Maleinsäure enthält. Dieses wird der Entsorgung zugeführt.

Das mit Acrylsäure beladene Extrakt / zweite Lösungsmittel Dimethylphthalat wird nach Erwärmung der erfindungsgemäßen Strippkolonne K8 am Kopf aufgegeben, und mit dem Kreisgas 8 wird die Acrylsäure ausgestrippt. Die für die Ausstrippung benötigt Wärme wird im Sumpf über einen Lösungsmittelkreislauf über einen Sumpfwärmetauscher W3 zugeführt.

Die Verwendung des Kreisgases, das für die Abtrennung von Essigsäure sowie von Acrolein und weiteren Bestandteilen im Verfahren bereits verwendet wird, bietet den Vorteil, dass das Verfahren hierauf stofflich ausgelegt ist und es keine Beeinflussung des Verfahrens hervorruft.

Da in der vierten Verfahrensvariante die Diacrylsäure-Konzentration im Sauerwasser höher ist und diese sich im Sumpf der Strippkolonne K8 aufpegelt, wird erfindungsgemäß zusätzlich noch eine Zersetzungsreaktion im Sumpf der Strippkolonne K8 initiiert. Diese Reaktion setzt bei Temperaturen > 165°C ein. Zur nahezu vollständigen Rückspaltung werden Verweilzeiten des zweiten Lösungsmittels bei diesen Temperaturen von mehr als 10 min, bevorzugt mehr als 30 min benötigt. Durch die Fahrweise der Strippkolonne K8 wird die in dem Strom des zweiten Lösungsmittels 5 im Sumpf ebenfalls enthaltene Diacrylsäure in monomere Acrylsäure rückgespalten und über den Kreisgasstrom 9 dem Verfahren wieder zugeführt.

Durch die Zersetzung der Diacrylsäure steigt die Gesamtmenge an rückgeführter Acrylsäure erfindungsgemäß an. Die Gesamtmenge des Strippgases bzw. des Kreisgases ist daher größer als bei den entsprechenden Kolonnen der dritten Verfahrensvariante, was sich auf die geometrische Auslegung der erfindungsgemäßen Strippkolonne K8 auswirkt.

Dem Sumpf der Strippkolonne K8 wird an Acrylsäure und Diacrylsäure abgereichertes zweites Lösungsmittel Dimethylphthalat entzogen, während am Kopf der Strippkolonne K8 das Kreisgas 9 mit einer signifikanten Menge Acrylsäure abgezogen wird.

Der mit der rückgewonnenen Acrylsäure beladene Kreisgasstrom 9 aus der Strippkolonne K8 wird dem Strippkreisgaswäscher K5 zugeführt. Dadurch bleibt die Gasbelastung dieser Kolonne im Vergleich zum Betrieb ohne die erfindungsgemäße Sauerwasserextraktion nahezu unverändert. Da die Temperatur des Kreisgasstroms 9 jedoch deutlich höher als in der herkömmlichen vierten Verfahrensvariante ist, wird erfindungsgemäß eine Kühleinrichtung W1 vorgeschaltet, mit welcher der erste Lösungsmittelstrom 10 vor Eintritt in den Stripkreisgaswäscher K5 vorgekühlt wird. Ohne diese Vorkühlung wäre unter Umständen mit einem Anstieg an Nebenkomponenten im Produkt zu rechnen. Sollten im weiteren Betrieb trotzdem signifikante Mengen an Nebenkomponenten im Produkt auftreten, kann dem durch eine weitere Absenkung der Temperatur des ersten Lösungsmittelstroms 10 entgegengesteuert werden, beispielsweise durch Einsatz von Kaltwasser anstelle von Kühlwasser in der Kühleinrichtung W1.

Als Grundlage für die Auslegung der Anlage wurde das Design der Strippkolonne wie sie bei der dritten Verfahrensvariante eingesetzt wird, verwendet. Diese nun erfindungsgemäße Strippkolonne K8 wurde mit neun Gleichgewichtsstufen und einer Sumpfspezifikation, also der Reinheit von rückgeführtem Lösungsmittel, mit 1385 ppm Acrylsäure ausgelegt. Um das Auslegungsrisiko so gering wie möglich zu halten, wurde der Kreisgasstrom durch die Strippkolonne K8 so weit erhöht, bis die Acrylsäurekonzentration am Kopf der Strippkolonne K8 im in den Prozess zurückgeführten Kreisgas so hoch wie im der dritten Verfahrensvariante war, das heißt, ca. 5 Gew.-%). Somit ergibt sich überraschenderweise ein ähnliches Konzentrationsprofil in der Strippkolonne K8 wie in der dritten Verfahrensvariante, so dass die Auslegung auf Basis der schon bestehenden Kolonnen mit geringem Risiko durchgeführt werden kann.

Grundsätzlich ist das Risiko für den Betrieb der Anlage für das erfindungsgemäße Verfahren relativ gering, da die gesamte erfindungsgemäße Sauerwasserextraktion im Bypass umgangen werden kann und somit jederzeit der Zustand vor der erfindungsgemäßen Änderung wiederhergestellt werden kann.

### Beispiel:

In einer technischen Anlage, die nach der vierten Verfahrensvariante arbeitet, wurden 250 t/h eines Reaktionsgases mit einer Temperatur von 265 °C in einen Direktgaskühler gefahren. Die Zusammensetzung des Gases betrug:

| | |
|---|---|
| Acrylsäure | 11,6 Gew.-% |
| Wasser | 5,0 Gew.-% |
| O₂, N₂, CO, CO₂ | 82,3 Gew.-% |
| Propen, Propan, Acrolein | 0,4 Gew.-% |
| Essigsäure | 0,3 Gew.-% |
| andere Carbonsäuren und Aldehyde | 0,4 Gew.-% |

In einer Absorptionsstufe K2 wurde das Gas einer Gegenstromabsorption unterworfen, bei der 138 t/h eines Lösungsmittelgemisches (erstes Lösungsmittel 3) mit einer Temperatur von 59 °C und einer Zusammensetzung von

| | |
|---|---|
| Diphyl | 70,3 Gew.-% |
| Dimethylphthalat | 19,7 Gew.-% |
| Acrylsäure | 2,8 Gew.-% |
| Diacrylsäure | 3,1 Gew.-% |
| Essigsäure | 0,4 Gew.-% |
| Phthalsäureanhydrid | 0,7 Gew.-% |
| Maleinsäureanhydrid | 0,4 Gew.-% |
| Wasser | 0,4 Gew.-% |
| andere Carbonsäuren, Aldehyde und Inhibitoren | 2,2 Gew.-% |

in den oberen Teil des Absorptionsteil der Absorptionsstufe K2 aufgegeben wurde.

Im oberen Teil der Absorptionsstufe K2 wurde das Reaktionsgas weiter auf 28 °C abgekühlt. Dabei wurde aus dem Kondensationsteil ein Sauerwasserstrom von 15,5 t/h mit einer Temperatur von 44 °C und einer Zusammensetzung von

| | |
|---|---|
| Wasser | 64,1 Gew.-% |
| Diphyl | 11,0 Gew.-% |
| Acrylsäure | 10,5 Gew.-% |
| Diacrylsäure | 0,2 Gew.-% |
| Essigsäure | 6,7 Gew.-% |
| Dimethylphthalat | 2,7 Gew.-% |
| Ameisensäure | 0,7 Gew.& |
| Maleinsäure | 1,0 Gew.-% |
| Andere Carbonsäuren und Aldehyde | 3,1 Gew.-% |

erhalten und einem Phasentrennbehälter B1 zugeführt. Vor Eintritt in den Phasentrennbehälter B1 wurde zur Vorextraktion 16,2 t/h erstes Lösungsmittel, welches aus dem Sumpf der Strippkreisgaswäsche K5 entnommen wurde, in das Sauerwasser 4 eingemischt. Das im Phasentrennbehälter B1 abgeschiedene Lösungsmittel wurde nach Vorextraktion in den Absorptionsteil der Absorptionsstufe K2 mit einem Mengenstrom von 18,3 t/h geleitet.

Aus dem Absorptionsteil der Absorptionsstufe K2 wurden ein Gemisch aus erstem Lösungsmittel mit Acrylsäure bei einer Temperatur von 114 °C und einer Zusammensetzung von

| | |
|---|---|
| Diphyl | 48,6 Gew.-% |
| Dimethylphthalat | 13,3 Gew.-% |
| Acrylsäure | 33,4 Gew.-% |
| Diacrylsäure | 1,9 Gew.-% |
| Maleinsäure | 0,7 Gew.-% |
| Wasser | 0,2 Gew.-% |
| Essigsäure | 0,2 Gew.-% |
| andere Carbonsäuren, Aldehyde Inhibitoren | 1,7 Gew.-% |

abgezogen, wovon 57,8 t/h in den Sumpf der Absorptionsstufe K2 und 229 t/h in eine Leichtsiederabtrennung geleitet wurden.

Nach der Passage der Leichtsiederabtrennung wurde der mit Acrylsäure beladene erste Lösungsmittelstrom 10 einer Vakuumdestillation unterworfen, wobei als Produkt 30 t/h Acrylsäure mit einer Reinheit von 99,7 Gew.-% am Seitenabzug der Reinkolonne abgezogen wurde.

Aus der Reinkolonne wurde am Sumpf ein an Acrylsäure abgereicherter Strom des ersten Lösungsmittels von 152,7 t/h bei einer Temperatur von 181 °C entnommen und nach Abkühlung auf 53 °C zum Kopf der Strippkreisgaswäsche K5 geleitet. Das abgereicherte Lösungsmittel hatte folgende Zusammensetzung:

| | |
|---|---|
| Diphyl | 71,8 Gew.-% |
| Dimethylphthalat | 20,0 Gew.-% |
| Acrylsäure | 1,7 Gew.-% |
| Diacrylsäure | 3,2 Gew.-% |
| Phthalsäureanhydrid | 0,7 Gew.-% |
| Maleinsäure | 0,8 Gew.-% |
| andere Carbonsäuren, Aldehyde Inhibitoren | 1,8 Gew.-% |

Nach Vorextraktion und Phasentrennung wurde aus dem Behälter B1 mit einem Volumen von 30 m³ ein Sauerwasserstrom von 15 t/h mit einer Zusammensetzung von

| | |
|---|---|
| Wasser | 76,3 Gew.-% |
| Acrylsäure | 9,6 Gew.-% |
| Diacrylsäure | 2,5 Gew.-% |
| Essigsäure | 6,6 Gew.-% |
| Dimethylphthalat | 0,5 Gew.-% |
| Ameisensäure | 0,7 Gew.-% |
| Maleinsäure | 1,8 Gew.-% |
| Andere Carbonsäuren und Aldehyde | 2,0 Gew.-% |

in die Sauerwasser-Extraktionskolonne K7 bei einer Temperatur von eingeleitet. Die Sauerwasser-Extraktionskolonne K7 hatte einen Durchmesser von 0,9 m und war über 34 m Länge mit einer strukturierten Packung B1-350 der Firma Montz gepackt. Die Packung war mittels vertikalen kreuzförmig angeordneten Trennblechen über die gesamte Länge in vier gleich große Kreissegmente unterteilt. Zur Reduktion der Fremdphase im Raffinat befand sich eine Schüttung von 300 m Länge aus 25 mm Pallringen im oberen Phasentrennbereich. Das Volumen des oberen und unteren Phasentrennbereichs betrug 3 m³

Zur Extraktion der im Sauerwasser enthaltenen Acrylsäure und Diacrylsäure wurde ein zweites Lösungsmittel 5, welches im Wesentlichen aus Dimethylphthalat bestand, mit einer Menge von 15 t/h bei einer Temperatur von 55 °C auf den Kopf der Extraktionskolonne K7 gegeben und dort gleichmäßig über den Querschnitt der Kolonne K7 verteilt. Die Extraktionskolonne K7 verließ ein an Acrylsäure abgereicherter Sauerwasserstrom von 12 t/h mit einer Zusammensetzung von

| | |
|---|---|
| Wasser | 89,5 Gew.-% |
| Essigsäure | 3,7 Gew.-% |
| Acrylsäure | 0,8 Gew.-% |
| Diacrylsäure | 0,1 Gew.-% |
| Dimethylphthalat | 0,9 Gew.-% |
| Maleinsäure | 2,3 Gew.-% |
| andere Carbonsäuren und Aldehyde | 2,7 Gew.-% |

Dieses Sauerwasser wurde in eine Anlage zur thermischen Behandlung zugeführt. Da in dem der Entsorgungseinheit zugeführten Raffinatstrom 11 noch ein Restanteil von Acrylsäure und Diacrylsäure befand, betrug der Verlust an Wertprodukt 108 kg/h oder 0,36 % bezogen auf die Produktion von 30 t/h.

Nach Phasentrennung am Boden der Extraktionskolonne K7 wurde das Extrakt in einer Menge von 18 t/h mit einer Zusammensetzung von

| | |
|---|---|
| Wasser | 3,7 Gew.-% |
| Essigsäure | 3,1 Gew.-% |
| Acrylsäure | 7,6 Gew.-% |
| Diacrylsäure | 2,3 Gew.-% |
| Dimethylphthalat | 80,9 Gew.-% |
| Diphyl | 0,4 Gew.-% |
| andere Carbonsäuren und Aldehyde | 2,0 Gew.-% |

nach Kreuzwärmetausch im Kreuzwärmetauscher W2 bei einer Temperatur von 110 °C auf den Kopf der Strippkolonne K8 gefahren. Die Strippkolonne K8 hatte eine Länge von 16,3 m und hatte einen über die Länge einheitlichen Innendurchmesser von 2,6 m. Die Kolonne K8 war im unteren Teil mit 5 DualFlow Böden und im oberen Teil mit 15 Ventilböden ausgerüstet. Der Bodenabstand im Bereich der DualFlow Böden betrug 600 mm und im Ventilbodenbereich 500 mm. Der Sumpfbereich der Kolonne K8 hatte zur Aufnahme des Sumpfprodukts ein Volumen von 14 m³.

Das Strippgas wurde in einer Menge von 30 t/h mit einer Temperatur von 130 °C in den Sumpf der Strippkolonne K8 gefahren und entsprach in seiner Zusammensetzung nach Verdichtung in einem Kreisgasverdichter dem Gas, welches am Kopf der Absorptionsstufe K2 erhalten wurde.

| | |
|---|---|
| Acrylsäure | 0,2 Gew.-% |
| Wasser | 1,9 Gew.-% |
| O₂, N₂, CO, CO₂ | 97,4 Gew.-% |
| Propen, Propan, Acrolein | 0,3 Gew.-% |
| Essigsäure | 0,1 Gew.-% |
| andere Carbonsäuren und Aldehyde | 0,1 Gew.-% |

Das Sumpfprodukt der Strippkolonne K8 würde über einen dampfbeheizten Rohrbündelwärmetauscher auf 190 °C erwärmt und über den Boden 5 im Kreis geführt. Der Flüssigkeitsinhalt des Rohrleitungssystem mit Wärmetauscher, über den die Flüssigkeit gekreist wurde, betrug 2,5 m³.

Das an Acrylsäure und Diacrylsäure abgereicherte Sumpfprodukt der Kolonne K8 wurde nach indirektem Wärmetausch in den Wärmetauschern W2 und W4 auf 55 °C abgekühlt und wieder auf den Kopf der Extraktionskolonne K7 aufgegeben. Das in die Extraktionskolonne K7 eintretende zweite Lösungsmittel hatte folgende Zusammensetzung:

| | |
|---|---|
| Dimethylphthalat | 97,1 Gew.-% |
| Diphyl | 0,4 Gew.-% |
| Acrylsäure | 0,1 Gew.-% |
| Diacrylsäure | 0,4 Gew.-% |
| Benzoesäure | 1,0 Gew.-% |
| Phthalsäure/Phthalsäureanhydrid | 0,6 Gew.-% |
| Maleinsäure/Maleinsäureanhydrid | 0,3 Gew.-% |
| Phenothiazin | 0,1 Gew.-% |

### Vergleichsversuch:

Die Einstellungen in der Synthese und in der Aufarbeitung wurden beibehalten und das Sauerwasser, welches den Behälter B1 verließ, wurde an der Sauerwasserextraktion bestehend aus den Kolonnen K7 und K8 vorbeigeführt, direkt in die Entsorgungseinheit eingeleitet und das Strippkreisgas, welches vorher über die Kolonne K8 geführt wurde, wurde direkt in den Sumpf Strippkolonne K5 eingeleitet. Bei dieser Einstellung änderte sich die Zusammensetzung des Sauerwassers bis auf den Essigsäureanteil, der sich von 3,7 Gew.-% auf 3,4 Gew.-% änderte, nicht.

Unter diesen Bedingungen reduzierte sich die Produktion auf 28,6 t/h. Der Verlust an Acrylsäure und Diacrylsäure betrug 1,8 t/h oder 6,2% der Produktion.

### Bezugszeichenliste

- 1: Reaktionsgemisch
- 3: erstes Lösungsmittel
- 4: Sauerwasser
- 4*: wässrige Phase des Sauerwassers 4
- 5: zweites Lösungsmittel
- 6: Extrakt
- 7 8: Kreisgas
- 9: mit Acrylsäure beladenes Kreisgases
- 10: erster Lösungsmittelstrom
- 11: Abwasserstroms
- 12: Teilstrom des ersten Lösungsmittelstroms 10
- 13: Kreisgas
- B1: Phasentrennbehälter
- K1: Kolonne K1
- K2: Absorptionsstufe
- K3: Kolonne K3
- K4: Kolonne K4
- K5: Strippkreisgaswäscher
- K6: Kolonne K6
- K7: Extraktionsstufe
- K8: Strippkolonne
- KB1: Kaminboden 1
- KB2: Kaminboden 2
- KB3: Kaminboden 3

## Patentansprüche

1. Verfahren zur Rückgewinnung von Acrylsäure, welche durch katalytische Gasphasenoxidation von Propen erhalten wird,
wobei in einer Absorptionsstufe (K2) die Acrylsäure aus dem Reaktionsgemisch (1) der Gasphasenoxidation mit einem ersten Lösungsmittel (3) absorbiert und zur destillativen Reinigung abgezogen sowie ein Gasgemisch aus der Absorptionsstufe (K2) zu einer Kondensationsstufe weitergeleitet wird,
wobei in der Kondensationsstufe das Gasgemisch kondensiert, eine kondensierte Phase des Gasgemisches als Sauerwasser (4) abgezogen und einer Phasentrennoperation in einem Phasentrennbehälter (B1) unterzogen wird, umfassend die Schritte
a) Zuführen der im Phasentrennbehälter (B1) gewonnenen wässrigen Phase (4*) des aus der Kondensationsstufe abgezogenen Sauerwassers (4) zu einer Extraktionsstufe (K7), in welcher in dem Sauerwasser (4) enthaltene Acrylsäure mit einem zweiten Lösungsmittel (5) extrahiert wird,
b) Zuführen des Acrylsäure enthaltenden Extrakts (6) zu einer Strippkolonne (K8), in welcher die Acrylsäure mit Kreisgas (8) aus dem zweiten Lösungsmittel (5) entfernt wird, wobei das abgetrennte zweite Lösungsmittel (5) nach Ausstrippung der Acrylsäure der Extraktionsstufe (K7) wieder zugeführt wird,
c) Zuführen des mit Acrylsäure beladenen Kreisgases (9) zu einem Strippkreisgaswäscher (K5), in welchem die Acrylsäure mit dem zu dem Strippkreisgaswäscher (K5) zugeführten ersten Lösungsmittelstrom (10) aus dem Kreisgas entfernt und in das erste Lösungsmittel (3) überführt wird, und
d) Zuführen des mit Acrylsäure beladenen ersten Lösungsmittels (3) zu einem ersten Teil zurück zu der Absorptionsstufe (K2).

2. Verfahren nach Anspruch 1, wobei der Extraktionsstufe (K7) eine Vorextraktionsstufe vorgeschaltet ist, in welcher dem Sauerwasser (4) ein Teilstrom (12) des aus dem Strippkreisgaswäscher (K5) austretenden ersten Lösungsmittelstroms zugeführt wird, nach Phasentrennung im Phasentrennbehälter (B1) das abgetrennte erste Lösungsmittel (3) der Absorptionsstufe (K2) zugeführt wird und das im Phasentrennbehälter (B1) abgetrennte Sauerwasser nach der Vorextraktion in der Vorextraktionsstufe zur Extraktionskolonne (K7) eingeleitet wird.

3. Verfahren nach Anspruch 1 oder 2, wobei die Absorptionsstufe (K2) mit einer Zulauftemperatur von 45 °C bis 65 °C und einer Ablauftemperatur von 100 °C bis 120 °C betrieben wird, wodurch das aus der Kondensationsstufe abgezogene Sauerwasser (4) neben einem erhöhten Gehalt an Acrylsäure ferner Diacrylsäure enthält.

4. Verfahren nach Anspruch 3, wobei in Schritt b) die Diacrylsäure in der Strippkolonne (K8) aufgespalten und auf diese Weise als monomere Acrylsäure rückgewonnen wird.

5. Verfahren nach einem der Ansprüche 1 bis 4, wobei dem Strippkreisgaswäscher (K5) im Strom des ersten Lösungsmittels (3) eine Kühleinrichtung (W1) vorgeschaltet ist, mit welcher das erste Lösungsmittel (3) vorgekühlt wird.

6. Verfahren nach einem der Ansprüche 1 bis 5, wobei die Strippkolonne (K8) bei einer Temperatur von mindestens 170 °C im Sumpf der Strippkolonne (K8) betrieben wird, bevorzugt bei 180 °C, besonders bevorzugt bei 190 °C, und die Verweilzeit der dem Sumpfkreislauf der Strippkolonne (K8) zugeführten Flüssigkeit > 0,5 h besonders bevorzugt > 1 h beträgt.

7. Verfahren nach einem der Ansprüche 1 bis 6, wobei das erste Lösungsmittel Diphyl enthält und das zweite Lösungsmittel Dimethylphthalat oder Diethylphthalat enthält.

8. Verfahren nach einem der Ansprüche 1 bis 7, wobei die Extraktion in der Extraktionskolonne (K7) bei Temperaturen von < 80 °C, bevorzugt 40 °C durchgeführt wird.

9. Anlage zur Rückgewinnung von Acrylsäure, welche eine Absorptionsstufe (K2) zur Absorption von Acrylsäure aus dem Reaktionsgemisch einer Gasphasenoxidation und eine daran anschließende Kondensationsstufe zur Kondensation des Gasgemischs als Sauerwasser (4) aufweist, ferner umfassend
- eine Extraktionsstufe (K7) zur Extraktion der in dem Sauerwasser (4) enthaltene Acrylsäure mit einem zweiten Lösungsmittel (5), wobei die Extraktionsstufe (K7) der Kondensationsstufe nachgelagert und mit dieser über eine Sauerwasser-Leitung verbunden ist,
- eine Strippkolonne (K5) zum Entfernen der Acrylsäure mit Kreisgas (13) aus dem zweiten Lösungsmittel (5), wobei die Strippkolonne (K8) der Extraktionsstufe (K7) nachgelagert und mit dieser über eine Extrakt-Leitung verbunden ist,
- eine Lösungsmittel-Leitung, welche die Strippkolonne (K8) und die Extraktionsstufe (K7) verbindet, zum Rückführen des zweiten Lösungsmittels (5) in die Extraktionsstufe (K7),
- einen Sumpfwärmetauscher (W3) im Sumpfkreislauf der Strippkolonne (K8) zum Bereitstellen der für das nahezu vollständige Ausstrippen der Acrylsäure aus dem Extraktstrom benötigten Energie,
- einen Strippkreisgaswäscher (K5) zum Entfernen der Acrylsäure mit einem zu dem Strippkreisgaswäscher (K5) zugeführten ersten Lösungsmittelstrom (10) aus dem Kreisgas, wobei der Strippkreisgaswäscher (K5) der Strippkolonne (K8) nachgelagert und mit dieser über eine Kreisgas-Leitung (9) verbunden ist, und
- eine erste Leitung zum Zuführen des mit Acrylsäure beladenen ersten Lösungsmittels (3) zu einem ersten Teil zurück zu der Absorptionsstufe (K1).

10. Anlage nach Anspruch 9, ferner umfassend
- eine Vorextraktionsstufe, die in der Sauerwasser-Leitung vor der Extraktionsstufe (K7) angeordnet ist.

11. Anlage nach Anspruche 9 oder 10, ferner umfassend
- eine Kühleinrichtung (W1) zum Vorkühlen des ersten Lösungsmittelstroms (10), wobei die Kühleinrichtung (W1) vor dem Strippkreisgaswäscher (K5) angeordnet ist.

12. Anlage nach einem der Ansprüche 9 bis 11, ferner umfassend
- einen Kreuzwärmetauscher (W2) zum Erwärmen des Extraktstroms vor Zulauf in die Strippkolonne (K8) bei gleichzeitigem Abkühlen des abgereicherten Extraktionsmittelstroms aus der Extraktionskolonne (K7).

13. Anlage nach einem der Ansprüche 9 bis 12, ferner umfassend
- einen Wärmetauscher (W4) zum Abkühlen des Extraktionsmittelstroms vor Eintritt in die Extraktionskolonne (K7).
